# EUROPEAN PATENT SPECIFICATION

(11) **EP 3 187 224 B1**
(45) Date of publication and mention of the grant of the patent: **02.06.2021**
(21) Application number: 16275002.0
(22) Date of filing: 04.01.2016
(51) Int. Cl.: A61N 1/375, A61N 1/36, A61N 1/362

(54) **ELECTRICAL CONNECTOR RING FOR IMPLANTABLE MEDICAL DEVICE**
ELEKTRISCHER VERBINDERRING FÜR IMPLANTIERBARE MEDIZINISCHE VORRICHTUNG
ANNEAU DE CONNECTEUR ÉLECTRIQUE POUR DISPOSITIF MÉDICAL IMPLANTABLE

(43) Date of publication of application: 05.07.2017
(73) Proprietor: Donatelle Plastics, Inc., New Brighton, Minnesota 55112 (US)
(72) Inventor: Vadlamudi, Raghu, Woodbury, Minnesota 55129 (US); Iwen, Matthew L., Savage, Minnesota 55378 (US); Fonder, Clint J., Elk River, Minnesota 55330 (US)
(74) Representative: Potter Clarkson

(56) References cited:
- US-A1- 2011 104 955
- US-A1- 2012 232 603
- US-A1- 2013 172 949
- US-B1- 7 299 095

## Description

### BACKGROUND OF THE INVENTION

### I. Field of the Invention

The present invention relates to implantable pulse generators and, more particularly, implantable pulse generator headers and the electrical connectors used in the construction of such headers. The present invention also relates to methods for manufacturing electrical connectors for headers for implantable pulse generators.

### II. Discussion of Related Art

In medical technology an implanted pulse generator (IPG) may be employed for a variety of purposes. An IPG is a battery powered device designed to deliver electrical stimulation to the body. An IPG is typically an integral component of a surgically implanted system, which includes the IPG, one or more leads and an external programmer. Such systems fall into two broad categories, neuromodulation systems and cardiac rhythm management systems. Neuromodulation systems are used, for example, to provide deep brain stimulation, vagus nerve stimulation, spinal cord stimulation, peripheral nerve stimulation and the like. Such stimulation has proven to be beneficial for the treatment of intractable pain, Parkinson's disease, pelvic disorders and incontinence, sleep apnea, and epilepsy, among other conditions. Cardiac rhythm management systems include heart pacemakers, defibrillators, cardioverters and other forms of devices used to monitor and control heart rhythms.

The IPG is typically implanted within a person's body, usually beneath the clavicle. Leads are then routed through the body between the site to be stimulated and the IPG. The leads are then coupled to the header of the IPG to carry signals between the IPG and the treatment site. The IPG can be calibrated using the external programmer by a physician (such as an electrophysiologist, neurologist or cardiologist) or by a nurse or other trained technician to meet the individual patient's needs. The IPG must be replaced periodically upon battery depletion. Battery depletion can occur within three to five years, though battery life is dependent on individual usage. End of battery life can be reasonably predicted by the use of telemetry between the IPG and the external programming device. This allows the IPG to be replaced prior to battery failure.

As indicated above, one example of an IPG is a heart pacemaker (or artificial heart pacemaker, so as not to be confused with the heart's natural pacemaker), a medical device which uses electrical impulses to regulate the beating of the heart. When the IPG is employed as an artificial heart pacemaker, the IPG is used in combination with a lead comprising a set of electrodes which carry stimulation pulses from the IPG to the heart and electrical signals back from the heart to the IPG which senses and responds to such signals. The primary purpose of a pacemaker is to maintain an adequate heart rate, either because the heart's native pacemaker is not fast enough, or because there is a block in the heart's electrical conduction system. Modern pacemakers are externally programmable and allow the electrophysiologist to select the optimum pacing modes for individual patients. Some IPG devices combine a pacemaker and defibrillator in a single implantable device. Multiple electrodes stimulating differing positions within the heart are often used to improve synchronization of the contractions of the upper and lower and chambers of the heart.

Another type of IPG is an implantable cardioverter-defibrillator (ICD), a small battery-powered electrical pulse generator which is implanted in patients who are at risk of sudden death due to ventricular fibrillation or ventricular tachycardia. The device is programmed to detect cardiac arrhythmia and correct it by delivering a jolt of electricity. In current variants, ICD devices have the ability to treat both atrial and ventricular arrhythmias as well as the ability to perform biventricular pacing in patients with congestive heart failure or bradycardia.

The process of implantation of an ICD is similar to implantation of a pacemaker. Like pacemakers, ICD devices are coupled to a set of leads containing electrode(s) and wire(s) which are passed though the vasculature to desired locations in the heart. For example an electrode can be passed through a vein to the right chambers of the heart, and then lodged in the apex of the right ventricle. Providing defibrillation pulses at this location has been found to be advantageous. As is the case with pacemaker leads, the leads are coupled to the header of the ICD and used to carry both stimulation pulses from the ICD to the heart and electrical signals from the heart to the ICD.

ICDs constantly monitor the rate and rhythm of the heart and can deliver therapies, by way of an electrical shock, when the electrical manifestations of the heart activity exceed one or more preset thresholds. More modern devices can distinguish between ventricular fibrillation and ventricular tachycardia (VT) and may try to pace the heart faster than its intrinsic rate in the case of VT, to try to break the tachycardia before it progresses to ventricular fibrillation. This is known as fast-pacing, overdrive pacing or anti-tachycardia pacing (ATP). ATP is only effective if the underlying rhythm is ventricular tachycardia, and is never effective if the rhythm is ventricular fibrillation.

Other IPG devices serve as neurostimulators and are used to treat pain, incontinence, and other neurologic and muscular conditions. Such IPG devices have a header used to couple the IPG to leads containing a plurality of wires and electrodes which deliver stimulating pulses from the IPG to nerves and muscles to provide beneficial therapies. The electrodes and wires of the leads may also be used to carry electrical signals back to the IPG.

The various types of IPG devices referenced above typically have a header to which the leads are attached. The header typically includes one or more bores each configured to receive a terminal pin of a lead. The terminal pin will typically contain a plurality of electrodes spaced along its length. Likewise, the bore will typically have a matching set of electrical contacts along its length which are spaced to form electrical connections with the electrodes of the lead pin. The electrical connections should be isolated from each other to prevent a short or unintended propagation of signals along a particular channel. The number and spacing or the electrodes and contacts may vary, but standards have emerged related to such numbers and such spacing for various types of stimulation systems.

Various types of electrical contacts have been employed in the headers of IPG devices. Prior art header designs often employed thin wire connections, female leaf springs, canted coil springs or "slide by" wire connectors. Many of these provided adequate electrical connection, but were fragile in design. Such connectors were easily damaged during pin insertion or incapable of producing mechanical forces sufficient to hold the pin in the desired orientation.

U.S. Patent No. 4,934,367 granted to Daglow et al on June 19, 1990 discloses the use of elastomeric rings either made of a conductive polymer or a non-conductive polymer impregnated with a conductive material. This and other patents also disclose the use of springs. For example, U.S. Patent No. 6,895,276 granted to Kast et al on May 17, 2005 discloses a contact comprising a cylindrical housing comprising a wall having an inner surface defining a bore, a channel between the inner surface of the wall and the bore and a continuous spring fitted within the channel. U.S. Patent No. 7,003,351 granted to Tvaska et al on February 21, 2006 and U.S. Patent 7,587,244 granted to Olbertz on September 8, 2009 each show a connector with a similar ring having a plurality of spring contact members attached thereto. Further, U.S. Patent 8,666,494 granted to Schramm et al discloses a spring contact ring comprising a housing including a recess channel and a spring comprising a base and a plurality of spring fingers. US 2011/0104955 describes a header, for an implantable medical device, having: a plurality of adjacent contact modules each having at least one lead passageway; a plurality of contact isolators, with a contact isolator of the plurality being disposed within each of the at least one lead passageways of the corresponding contact modules of the plurality; and a plurality of electrical contacts positioned within the lead passageway, with at least one electrical contact of the plurality being located axially between contact isolators, each of the plurality of contacts being in electrical communication with a corresponding pin of the feedthrough. US 2013/0172949 describes an apparatus, for use within a header of an implantable medical device, including a substantially annular spring, sized and shaped to be disposed in the header, the spring defining a loop extending about a central axis, the spring including a plurality of elastically deformable switchback portions that both zigzag and curve transversely about the loop to define a surface that at least partially encompasses the loop.

Designing a connector for use in the header of an IPG device presents a variety of challenges which arise from the difficulty in maintaining the desired balance between mechanical and electrical properties. Examples of such challenges include: (1) limiting the mechanical insertion force required to insert the lead pin because excessive pressure exerted on the inner seal and electrical components of the bore can result in damage to the header; (2) excessive electrical engagement between the contacts of the header and the lead pin can result in shorts or faults which can draw off potential battery power; (3) insufficient mechanical retention forces can result in an electrode of the bore losing position or falling out of place; and (4) maintaining proper manufacturing tolerances. The tolerances of the electrode lead wires present further challenges with respect to the header's ability to achieve the desired electrical and mechanical responses. Thus, there continues to exist a real and substantial need to provide efficient and cost effective manufacturing methods and electrical contact designs for headers which meet these challenges.

### SUMMARY OF THE INVENTION

The aforementioned problems are solved by providing a ringlet coupler according to claim 1, which can be employed in the header of an implantable pulse generator and which is adapted to make an electrical connection with a contact of a lead pin of a medical lead. The components of the ringlet coupler can be inexpensively and quickly fabricated using high speed machining operations. Only a few assembly steps are required.

The components include a housing having a wall defining an inner surface and an outer surface. The components also include an electrically conductive ringlet. The band also has an inner surface and an outer surface and is fabricated into a pattern consisting of at least three smoothly curved foils joined together by smoothly curved reduced diameter sections between each adjacent pairs of foils. The reduced diameter sections define a channel and the foils are equally spaced about this channel. When the ringlet is placed in the housing, the apex of each foil is in contact with the inner surface of the housing. When a lead pin is inserted into the channel, the reduced diameter sections simultaneously engage the lead pin to form an electrical connection. Likewise, the reduced diameter sections cooperate with the foils and the housing to mechanically secure the lead pin to the electrically conductive ringlet. One or more supports may be employed to secure the ringlet to the inner surface of the housing.

Electrical contact and the mechanical holding force of the ringlet are typically improved by providing a concave area of reduced thickness at each of the reduced diameter sections. These concave areas extend inwardly from the second inner surface of the band. Similar concave areas of reduced thickness may be provided at the apex of the foils. These reduced diameter sections serve other important functions as well. They permit flexing of the ringlet which assists with assembly of the ringlet and the housing. Such flexing is also advantageous to permit insertion and withdrawal of the lead pin. Such flexing is further assisted by providing a break at the apex of one of the foils.

The housing and the ringlet can be made from a wide variety of materials. Conductive alloys suitable for use when forming the ringlet include a nickel, cobalt, chromium and molybdenum alloy made in conformance with ASTM F562-13 which is widely used for surgical implants. This alloy is known for its strength and ductility. Other suitable materials include conductive thermoplastics, combinations of thermoplastics and metals, and other metals such as platinum-iridium, iron, titanium, gold, silver, copper and various conductive alloys.

The number of foils of the ringlet can be increased beyond three. In addition to a trefoil shape, other shapes (such as a quatrefoil shape, a pentafoil shape or a hexafoil shape) may be employed.

Assembly of the ringlet to the housing can occur in several different ways. For example, the ringlet may be inserted through one of the two open ends of the housing. Alternatively, a slot may be provided through the wall of the housing and the ringlet can then be inserted through this slot.

Various supports may be employed to secure the ringlet in place to the inner surface of the housing. For example, a recessed channel defined by a pair of walls extending from the inner surface of the ring may be employed. The ringlet may be positioned in this channel such that the two channel walls extend on opposite sides of the ringlet. Alternate supports may be employed to couple the ringlet in place with the housing. The housing may also have an internal wall which prevents the ringlet from being pushed all the way through the housing. A cap may also be employed such that the ringlet is sandwiched between the internal wall and cap within the housing.

### DESCRIPTION OF DRAWINGS

Figure 1 is a perspective exploded view of a ringlet coupler wherein the ringlet has a trefoil shape;
Figure 2 is an end view of the ringlet coupler of Figure 1;
Figure 3 is a perspective view of an alternative embodiment of a ringlet coupler wherein the ringlet has a hexafoil shape;
Figure 4 is an end view of the ringlet coupler of Figure 3;
Figure 5 is a perspective view of an alternative embodiment of a ringlet coupler having a hexafoil shape;
Figure 6 is an end view of the ringlet coupler of Figure 5;
Figure 7 is a plan view of a quatrefoil shaped ringlet; and
Figure 8 is a plan view of a pentafoil shaped ringlet.

### DESCRIPTION OF PREFERRED EMBODIMENT

The following discussion is presented to enable a person skilled in the art to make and use the present teachings. Various modifications to the illustrated embodiments will be readily apparent to those skilled in the art, and the principles described herein may be applied to other embodiments and applications without departing from the present invention. Thus, the present invention is not intended to be limited to embodiments shown, but is to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the present invention. Skilled artisans will recognize many useful alternatives to the teachings and the examples provided herein falling within the scope of the appended claims exist and may be employed without deviating from the invention.

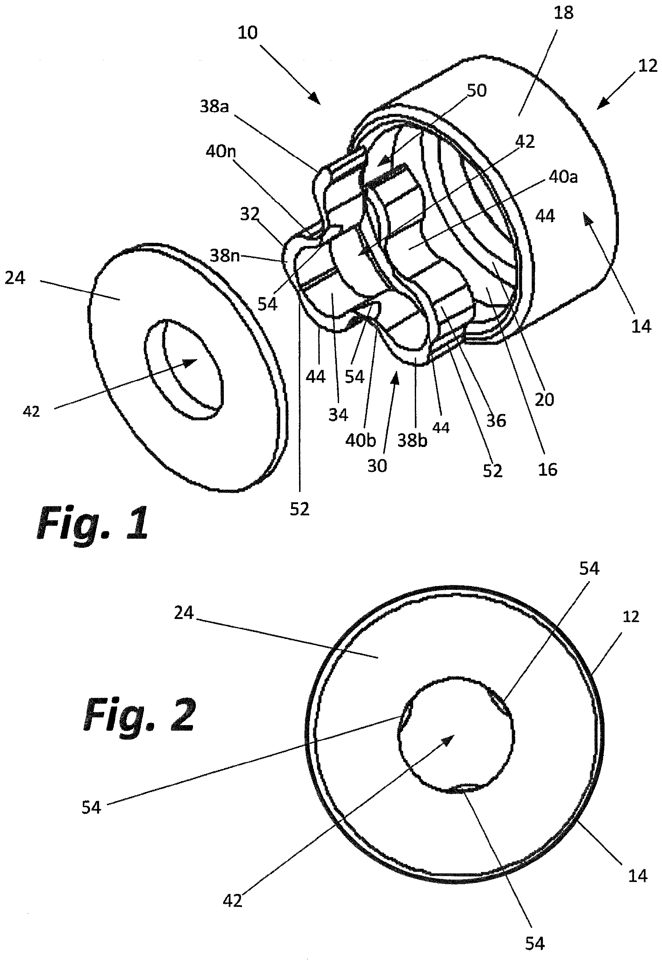

Figure 1 shows a ringlet coupler 10 comprising a housing 12 and a ringlet 30. The housing 12 has an outer cylindrical wall 14 with a first inner surface 16 and a first outer surface 18. Extending inwardly from the first inner surface 16 is a support or wall 20. The support 20 provides a seat against which the ringlet 30 rests to prevent the ringlet 30 from being pushed all the way through the housing 12. In this fashion, the wall 20 is a support adapted to secure the ringlet 30 to the inner surface 16 of the housing. The housing 12 also includes a cap 24. After the ringlet 30 is inserted into the housing, cap 24 is then inserted and fastened in place using a weld, adhesive or friction such that the ringlet is sandwiched between and supported between the cap 24 and wall 20, further securing the ringlet 30 to the inner surface 16 of the housing 12.

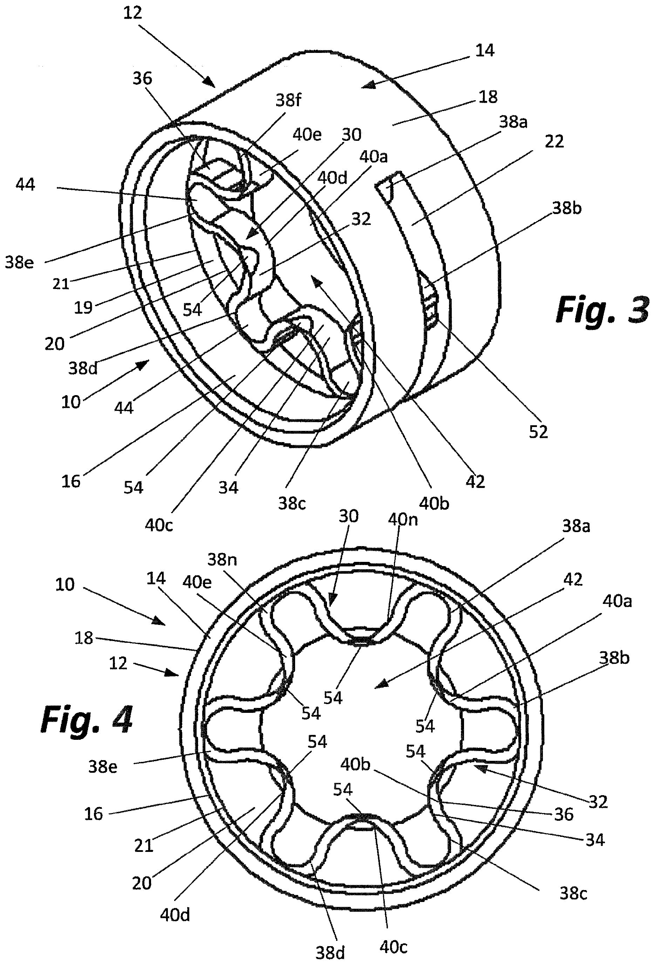

Various alterations may be made to the housing. As shown in Figure 3, the cylindrical wall 14 may be provided with a slot 22 through which the ringlet 30 may be inserted into the housing 12. As is also shown in Figure 3, rather than the housing 12 having a wall 20 and cap 24, the housing 30 may be provided with a channel 19 defined by walls 20 and 21. The channel 19 is aligned with slot 22 so that the ringlet 30 may be inserted through slot 22 and into the channel 19 to capture the ringlet 30 in the channel 19 between walls 20 and 21 which serve as a support to secure the ringlet 30 to the inner surface 16 of the housing.

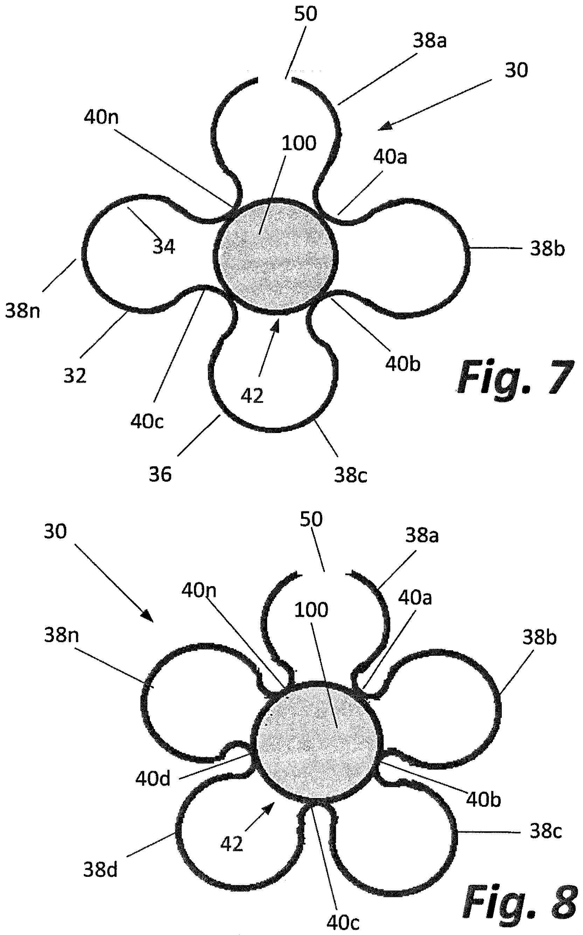

Figures 1-8 show alternative ringlets 30. In each embodiment, the ringlets 30 comprise a band 32. The band 32 has a second inner surface 34 and a second outer surface 36. The band is fabricated into smoothly curved foils 38a-n. The foils 38a-n are joined together by smoothly curved reduced diameter sections 40a-n. Figures 1 and 2 show a ringlet 30 as having a trefoil configuration while Figures 3-6 show a ringlet 30 having a hexafoil configuration. Figure 7 shows the ringlet 30 having a quatrefoil configuration and Figure 8 shows the ringlet having a pentafoil configuration. The number of foils is not critical so long as at least three foils are present.

Irrespective of the number of foils, 38a-n, they are preferably equally spaced about a channel 42 defined by the reduced diameter sections 40a-n. Channel 42 extends not only through the ringlet 30, but also through the housing 12 and any cap 24 which is employed. A lead pin 100 is inserted into the channel 42 to couple the lead to the ringlet coupler 10.

Each foil 38a-n has an apex 44. When assembled, the apex 44 of each foil 38a-n is adjacent to and typically is in contact with the inner surface 16 of the housing 12. When a pin 100 of a medical lead (shown in Figures 7 and 8) is inserted into the channel 42, the reduced diameter sections 40a-n simultaneously engage the lead pin 100 to form an electrical connection between the ringlet coupler 10 and the lead pin 100. Further, the reduced diameter sections 40a-n cooperate with the foils 38a-n and the housing 12 to mechanically secure the lead pin 100 to the ringlet 30.

The ringlet 30 is made of an electrically conductive material. The housing 12 may also be made of an electrically conductive material. If the housing 12 is electrically conductive, wires (not shown) extending to the circuitry of the pulse generator are coupled to the housing 12. Otherwise, such wires are coupled to the ringlet 30. The ringlet 30 may be made of any suitable conductive, biocompatible material. Alloys of cobalt, nickel, chromium and molybdenum made in conformance with ASTM International Standard F562-13 have proven to be well suited for use in medical implants generally. Such an alloy is also well suited for forming the ringlet 30. As discussed above, various thermoplastics, combinations of thermoplastics and metals, metals, and metal alloys may be used to fabricate the ringlet 30.

Irrespective of the number of foils, the ringlet 30 may also be provided with other desirable features. As shown in Figures 1, 7 and 8, the ringlet 30 includes a break 50. This break 50 enhances flexibility of the ringlet 30 which aid in insertion of the ringlet into the housing. This break 50 also allows the ringlet 30 to flex (without damage to the lead or ringlet coupler 10) as a lead pin is pushed into or pulled out of the channel 42. To further aid such flexibility and provide better attachment between the ringlet 30 and the cylindrical wall of the housing 12, the apex 44 of each foil 38a-n (other than the foil including the break 50) may be provided with a concave area 52 of reduced thickness extending inwardly from the second outer surface 36. To still further aid in providing such flexibility and increase the area of contact between the ringlet 30 and the lead pin 100, a concave area 54 of reduced thickness extending inwardly from the second inner surface 34 may be provided at each of the reduced diameter sections 40an.

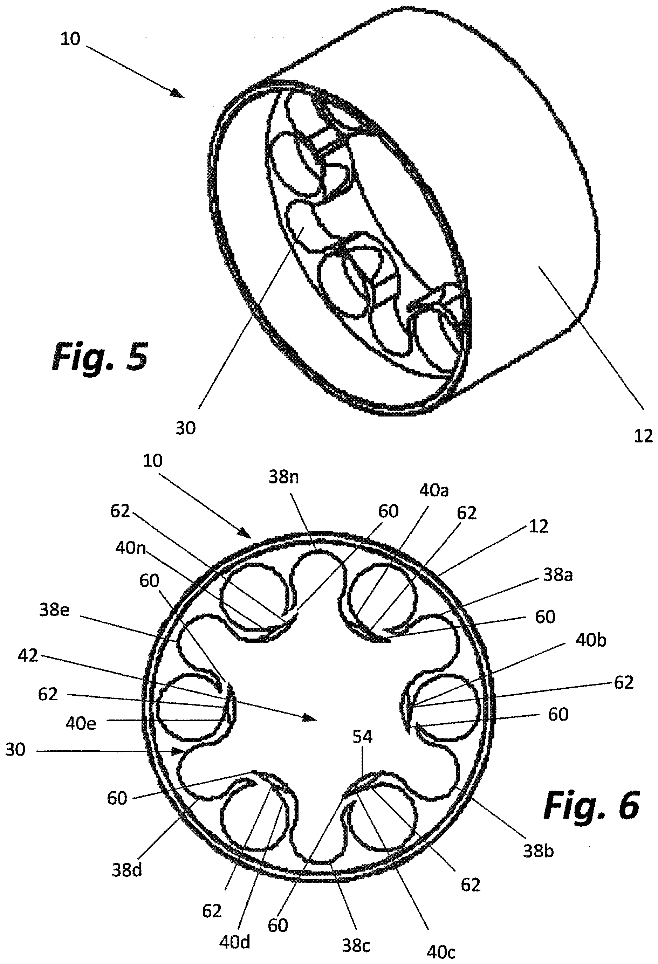

The embodiment of Figures 1 and 2 and the embodiment of Figures 3 and 4 each show a separately fabricated housing 12 and ringlet 30 which are coupled together. It is, however, possible to fabricate the housing 12 and ringlet 30 as a single piece. Figures 5 and 6 show such an embodiment. Specifically, housing 12 and ringlet 30 of the ringlet coupler 10, shown in Figures 5 and 6, are fabricated as a single piece, either as part of a cutting, molding or other fabrication process. This eliminates the need for the support wall 20 and cap 24 of the embodiment of Figures 1 and 2 or the need for the slot 22 and channel 19 defined by walls 20 and 21 in the embodiment of Figures 3 and 4.

Further, while the embodiment shown in Figures 5 and 6 has a hexafoil-shaped ringlet 30 like the embodiment shown in Figures 3 and 4, the reduced diameter sections 40a-n of the embodiment of Figures 5 and 6 differ because they each include a small gap 60 and a flap 62 which at least partially closes the gap 60 when a pin 100 is inserted into the channel 42. As such, the pin is resiliently engaged by the flaps 62.

As noted above, while Figures 1 and 2 show trefoil shaped ringlet 30 and Figures 3-6 show a hexafoil-shaped ringlet 30, other shapes may be employed such as the quatrefoil shape shown in Figure 7 and the pentafoil shape shown in Figure 8. To achieve proper electrical and mechanical contact with the lead pin 100, there must be at least three foils. Any reasonable number of additional foils may be employed.

Those skilled in the art will recognize that a plurality of the ringlet couplers 10 may be stacked with seals (not shown) separating the electrical connectors. Stacks of connectors 10 and seals may then be overmolded with silicone or some other suitable material to form the header of a pulse generator. See, for example, U.S. Patent No. 8,666,494 to Schramm et al, granted March 4, 2014, which is incorporated by reference and shows a stack of electrical connectors and seals overmolded to form the header of an implantable pulse generator. The lead pin 100 is used to couple the lead to the pulse generator.

The foregoing discussion of various embodiments of the invention is not intended to be limiting. They are instead intended to describe the invention in sufficient detail to enable one of ordinary skill in the art. The scope of the invention is only limited by the following claims.

## Claims

1. A ringlet coupler (10) configured to be employed in the header of an implantable pulse generator and adapted to make an electrical connection with a contact of a lead pin of a medical lead, said ringlet coupler comprising:
(a) an electrically conductive housing (12) having an outer cylindrical wall (14) defining a first inner surface (16) and first outer surface (18) with a pair of walls (20, 21) extending inwardly from the first inner surface (16) of the outer cylindrical wall (12), said pair of walls defining a first channel (19) aligned with a slot (22), said slot configured such that an electrically conductive ringlet (30) may be inserted through the slot (22) into the first channel (19) to capture the ringlet (30) in the first channel (19) between the walls (20, 21) to secure the ringlet (30) to the inner surface (16) of the housing (12),
(b) said electrically conductive ringlet (30) comprising a band (32), said band having a second inner surface (34) and a second outer surface (36), and fabricated into at least three smoothly curved foils (38a-n) joined together by smoothly curved reduced diameter sections (40a-n) between adjacent foils, wherein said foils are equally spaced about a second channel (42) defined by said reduced diameter sections (40a-n), wherein each foil has an apex (44) adjacent to the inner surface (16) of the housing (12), wherein the reduced diameter sections (40a-n) are adapted to simultaneously engage a lead pin (100) inserted into the second channel (42) to form an electrical connection and cooperate with the foils and the housing to mechanically secure such a lead pin to the electrically conductive ringlet (30).

2. The ringlet coupler (10) of claim 1 wherein said band (32) further comprises a concave area (54) of reduced thickness at each of the reduced diameter sections (40a-n) extending inwardly from the second inner surface (34).

3. The ringlet coupler (10) of claim 1 wherein said band (32) further comprises a break (50) at the apex (44) of a first of the foils (38a).

4. The ringlet coupler (10) of claim 3 wherein said band (32) further comprises a concave area (52) of reduced thickness extending inwardly from the second outer surface (36) at the apex (44) of each of the foils (38b-n) other than the first of the foils (38a).

5. The ringlet coupler (10) of claim 1 wherein said ringlet (30) is made of a conductive metal alloy.

6. The ringlet coupler of claim 5 wherein said conductive metal alloy comprises nickel, cobalt, chromium and molybdenum.

7. The ringlet coupler (10) of claim 1 wherein said ringlet (30) has a trefoil shape.

8. The ringlet coupler of claim 1 wherein said ringlet (30) has a shape selected from a group consisting of a quatrefoil shape, a pentafoil shape and a hexafoil shape.

9. The ringlet coupler (10) of claim 1 wherein a first of said foils (38a) has a break (50) at its apex (44), and said band (32) further comprises a concave area (52) of reduced thickness extending inwardly from the second outer surface (36) at the apex (44) of each of the foils (38b-n) other than the first of the foils (38a), wherein the concave area of reduced thickness extending inwardly from the second outer surface (36) at the apex (44) of each of the foils (38b-n) is adapted to engage the inner surface of the outer cylindrical wall (14).

## Patentansprüche

1. Ringkoppler (10), der konfiguriert ist, um in dem Kopf eines implantierbaren Impulsgenerators verwendet zu werden und angepasst ist, um eine elektrische Verbindung mit einem Kontakt eines Leitungsstifts einer medizinischen Leitung herzustellen, wobei der Ringkoppler Folgendes umfasst:
(a) ein elektrisch leitendes Gehäuse (12), das eine äußere zylindrische Wand (14) aufweist, die eine erste Innenoberfläche (16) und eine erste Außenoberfläche (18) mit einem Wandpaar (20, 21) definiert, das sich von der ersten Innenoberfläche (16) der äußeren zylindrischen Wand (12) nach innen erstreckt, wobei das Wandpaar einen ersten Kanal (19) definiert, der an einem Schlitz (22) ausgerichtet ist, wobei der Schlitz derart konfiguriert ist, dass ein elektrisch leitender Ring (30) durch den Schlitz (22) in den ersten Kanal (19) eingeführt werden kann, um den Ring (30) in dem ersten Kanal (19) zwischen den Wänden (20, 21) zu erfassen, um den Ring (30) an der Innenoberfläche (16) des Gehäuses (12) zu befestigen,
(b) wobei der elektrisch leitende Ring (30) ein Band (32) umfasst, wobei das Band eine zweite Innenoberfläche (34) und eine zweite Außenoberfläche (36) aufweist, und in wenigstens drei glatt gebogene Folien (38a-n) gefertigt ist, die durch glatt gebogene Abschnitte mit reduziertem Durchmesser (40a-n) zwischen angrenzenden Folien zusammengefügt sind, wobei die Folien um einen zweiten Kanal (42) herum gleichmäßig beabstandet sind, der durch die Abschnitte mit reduziertem Durchmesser (40a-n) definiert ist, wobei jede Folie einen Scheitelpunkt (44) aufweist, der angrenzend an die Innenoberfläche (16) des Gehäuses (12) ist, wobei die Abschnitte mit reduziertem Durchmesser (40a-n) angepasst sind, um einen Leitstift (100) gleichzeitig in Eingriff zu nehmen, der in den zweiten Kanal (42) eingeführt ist, um eine elektrische Verbindung auszubilden und mit den Folien und dem Gehäuse zusammenzuwirken, um einen solchen Leitungsstift an dem elektrisch leitenden Ring (30) mechanisch zu befestigen.

2. Ringkoppler (10) nach Anspruch 1, wobei das Band (32) ferner einen konkaven Bereich (54) mit verringerter Dicke an jedem der Abschnitte mit verringertem Durchmesser (40a-n) umfasst, der sich von der zweiten Innenoberfläche (34) nach innen erstreckt.

3. Ringkoppler (10) nach Anspruch 1, wobei das Band (32) ferner einen Bruch (50) an dem Scheitelpunkt (44) einer ersten der Folien (38a) umfasst.

4. Ringkoppler (10) nach Anspruch 3, wobei das Band (32) ferner einen konkaven Bereich (52) mit verringerter Dicke umfasst, der sich von der zweiten Außenoberfläche (36) an dem Scheitelpunkt (44) jeder der Folien (38b-n) anders als die erste der Folien (38a) nach innen erstreckt.

5. Ringkoppler (10) nach Anspruch 1, wobei der Ring (30) aus einer leitfähigen Metalllegierung hergestellt ist.

6. Ringkoppler nach Anspruch 5, wobei die leitfähige Metalllegierung Nickel, Kobalt, Chrom und Molybdän umfasst.

7. Ringkoppler (10) nach Anspruch 1, wobei der Ring (30) eine Kleeblattform aufweist.

8. Ringkoppler nach Anspruch 1, wobei der Ring (30) eine Form aufweist, die aus einer Gruppe ausgewählt ist, die aus einer Vierblattform, einer Fünfblattform und einer Sechsblattform besteht.

9. Ringkoppler (10) nach Anspruch 1, wobei eine erste der Folien (38a) an ihrem Scheitelpunkt (44) einen Bruch (50) aufweist und das Band (32) ferner einen konkaven Bereich (52) mit verringerter Dicke umfasst, der sich von der zweiten Außenoberfläche (36) an dem Scheitelpunkt (44) jeder der Folien (38b-n) anders als die erste der Folien (38a) nach innen erstreckt, wobei der konkave Bereich mit verringerter Dicke, der sich von der zweiten Außenoberfläche (36) an dem Scheitelpunkt (44) jeder der Folien (38b-n) nach innen erstreckt, angepasst ist, um die Innenoberfläche der äußeren zylindrischen Wand (14) in Eingriff zu nehmen.

## Revendications

1. Coupleur annulaire (10) conçu pour être utilisé dans l'embase d'un générateur d'impulsions implantable et adapté pour établir une connexion électrique avec un contact d'une broche conductrice d'un conducteur médical, ledit coupleur annulaire comprenant :
(a) un boîtier électriquement conducteur (12) ayant une paroi cylindrique extérieure (14) définissant une première surface intérieure (16) et une première surface extérieure (18) avec une paire de parois (20, 21) s'étendant vers l'intérieur à partir de la première surface intérieure (16) de la paroi cylindrique extérieure (12), ladite paire de parois définissant un premier canal (19) aligné avec une fente (22), ladite fente étant conçue de telle sorte qu'un anneau (30) électriquement conducteur puisse être inséré à travers la fente (22) dans le premier canal (19) pour capturer l'anneau (30) dans le premier canal (19) entre les parois (20, 21) pour fixer l'anneau (30) à la surface intérieure (16) du boîtier (12),
(b) ledit anneau (30) électriquement conducteur comprenant une bande (32), ladite bande ayant une seconde surface intérieure (34) et une seconde surface extérieure (36), et fabriquée en au moins trois feuilles légèrement incurvées (38a-n) reliées ensemble par des sections de diamètre réduit légèrement incurvées (40a-n) entre des feuilles adjacentes, lesdites feuilles étant également espacées autour d'un second canal (42) défini par lesdites sections de diamètre réduit (40a-n), chaque feuille ayant un sommet (44) adjacent à la surface intérieure (16) du boîtier (12), les sections de diamètre réduit (40a-n) étant adaptées pour venir en prise simultanément avec une broche conductrice (100) insérée dans le second canal (42) pour former une connexion électrique et coopérer avec les feuilles et le boîtier afin de fixer mécaniquement une telle broche conductrice à l'anneau (30) électriquement conducteur.

2. Coupleur annulaire (10) selon la revendication 1, dans lequel ladite bande (32) comprend en outre une zone concave (54) d'épaisseur réduite au niveau de chacune des sections de diamètre réduit (40a-n) s'étendant vers l'intérieur à partir de la seconde surface intérieure (34).

3. Coupleur annulaire (10) selon la revendication 1, dans lequel ladite bande (32) comprend en outre une cassure (50) au niveau du sommet (44) d'une première des feuilles (38a).

4. Coupleur annulaire (10) selon la revendication 3, dans lequel ladite bande (32) comprend en outre une zone concave (52) d'épaisseur réduite s'étendant vers l'intérieur à partir de la seconde surface extérieure (36) au niveau du sommet (44) de chacune des feuilles (38b-n) autre que la première des feuilles (38a).

5. Coupleur annulaire (10) selon la revendication 1, dans lequel ledit anneau (30) est constitué d'un alliage métallique conducteur.

6. Coupleur annulaire selon la revendication 5, dans lequel ledit alliage métallique conducteur comprend du nickel, du cobalt, du chrome et du molybdène.

7. Coupleur d'anneau (10) selon la revendication 1, dans lequel ledit anneau (30) a une forme de trèfle.

8. Coupleur annulaire selon la revendication 1, dans lequel ledit anneau (30) a une forme choisie dans un groupe constitué d'une forme quadrilobe, d'une forme à cinq feuilles et d'une forme hexalobée.

9. Coupleur annulaire (10) selon la revendication 1, dans lequel une première desdites feuilles (38a) a une cassure (50) à son sommet (44), et ladite bande (32) comprend en outre une zone concave (52) d'épaisseur réduite s'étendant vers l'intérieur à partir de la seconde surface extérieure (36) au niveau du sommet (44) de chacune des feuilles (38b-n) autre que la première des feuilles (38a), la zone concave d'épaisseur réduite s'étendant vers l'intérieur à partir de la seconde surface extérieure (36) au niveau du sommet (44) de chacune des feuilles (38b-n) étant adapté pour venir en prise avec la surface intérieure de la paroi cylindrique extérieure (14).
